# EUROPEAN PATENT APPLICATION

(11) **EP 2 229 835 A1**
(43) Date of publication of application: **22.09.2010**
(21) Application number: 09155724.9
(22) Date of filing: 20.03.2009
(51) Int. Cl.: A44C 5/00, A44C 15/00, A44C 25/00, A61L 9/12

(54) **Perfume diffusing jewelry**

(71) Applicant: Trendstar Nederland, 1185 PP Amstelveen (NL)
(72) Inventor: Theunisz, Johan André, 1185 PP Amstelveen (NL)
(74) Representative: Ketelaars, Maarten F.J.M.

(57) **Abstract**

The invention relates to a piece of jewelry, for use on a person's body, comprising a rigid casing forming a cavity, the cavity being arranged to hold a body of porous material suitable for absorbing and emitting a liquid fragrance, the casing comprises an opening and a sealing mechanism allowing the body of porous material to be removed from and inserted in the cavity, and the casing comprises one or more slots constituting aerial ducts between the cavity and the surroundings of the piece of jewelry, allowing the liquid fragrance to evaporate, with a body of porous material consisting of a ligneous absorbent.

## Description

### TECHNICAL FIELD

The present invention relates to a piece of jewelry.

### BACKGROUND

Manufacturing and use of perfume as an article of luxury has been a human activity for several thousands of years. Unfortunately for some people, an allergy or some other physical cause withholds them from applying perfume directly to their skin. For those people, small wearable containers for spreading perfume present an outcome. The integration of a perfume dispensing or spreading function into a piece of jewelry has been known for quite some time.

US patent number 7,073,729 describes many variants of jewelry pieces incorporating a rigid ceramic porous body. This porous body is designed as a decorative slab of material arranged to efficiently absorb a fragrance emitting liquid compound like perfume or eau de toilette on both sides. This porous body is attached firmly to a base, exposing the porous body to air. When worn, the generated body heat causes the perfume to evaporate and diffuse into the surrounding air.

Gebrauchsmusterschrift DE202004013535U1 discloses a similar piece of jewelry, featuring a perfume carrier composed of synthetic material.

In any case, jewelry can be considered as a durable commodity. Accordingly, one can expect the fragrance emission capability to be revitalized many times before a piece of jewelry wears out.

It would be beneficial to provide a piece of perfume emitting jewelry with improved emission characteristics and improved usability by facilitating the ease of replenishment or of replacement of the perfume contained by the porous body.

### SUMMARY

It is an object to provide a piece of jewelry which overcomes or reduces at least one of the disadvantages of the prior art.
Therefore, according to an aspect there is provided a piece of jewelry, for use on a person's body, comprising a rigid casing forming a cavity, the cavity being arranged to hold a body of porous material suitable for absorbing and emitting a liquid fragrance, the casing comprising an opening and sealing mechanism allowing the body of porous material inside the cavity to be accessed, and the casing comprising at least one slot, the at least one slot constituting an aerial duct between the cavity and the surroundings of the piece of jewelry, allowing the liquid fragrance to evaporate, wherein the body of porous material consists of a ligneous absorbent.

Advantageously, a body of ligneous porous material is cheap and easy to manufacture. If accompanied by a robust user friendly opening mechanism, this embodiment is easy to utilize and maintain. In an embodiment, the body of ligneous material may consist of balsa wood.

According to a further aspect there is provided a method for fixating the body of porous material inside the cavity of the piece of jewelry having a body axis that is substantially circular or helical, the method comprising:
(a) soaking the body of porous material with a volatile liquid prior to insertion into the cavity;
(b) inserting the soaked body of porous material through a tap hole into the cavity.

### BRIEF DESCRIPTION OF THE DRAWINGS

Embodiments will now be described, by way of example only, with reference to the accompanying schematic drawings, which are not necessarily drawn to scale and wherein:
Fig.'s 1a and 1b schematically show perspective views of two embodiments,
Fig. 2 presents a schematic cross-section of the embodiment shown in Fig. 1a,
Fig. 3 displays a pair of slots according to an embodiment, and
Fig.'s 4a and 4b depict perspective and exploded views of a toroidal bracelet.

### DETAILED DESCRIPTION

A piece of jewelry is provided with a body of porous material, such as a ligneous material for containing and emitting a liquid fragrance. In this text the liquid fragrance is referred to as perfume, although it could also represent eau de Cologne, eau de toilette or other similar volatile aromatic substances. One or more recesses are provided that penetrate the casing and permit the air to circulate between the cavity holding the body of porous material and the casing exterior, constituting a path for the perfume to diffuse to the surroundings. The recesses may also be designed to facilitate convection of the perfume to the surroundings and supply of fresh air into the casing. The shape of a recess may be a slot with an elongation in a direction along the surface of the casing. The perfume emitting mechanism is based on the body temperature of a user causing the liquid perfume contained by the porous body to evaporate. The body heat and movements of the user (causing convection) stimulates emission of the perfume. By optimizing the location and orientation of the slots, a desired rate of perfume emission can be achieved.

So, in general there is provided a piece of jewelry 1, 2, 3, for use on a person's body, comprising a rigid casing 4 forming a cavity 5, the cavity being arranged to hold a body of porous material 6 suitable for absorbing and emitting a liquid fragrance, the casing comprises an opening and sealing mechanism 8 allowing the body of porous material inside the cavity to be accessed, and the casing comprises at least one slot 10, the at least one slot constituting an aerial duct between the cavity and the surroundings of the piece of jewelry, allowing the liquid fragrance to evaporate, wherein the body of porous material consists of a ligneous absorbent.

The body of porous material 6 may consists of various types of wood. Wood is a readily available and relatively cheap raw material that is quite strong, considering its low mass density. Several types of wood have a porous structure. Such a material yields an absorbing porous body with a low weight, increasing the ease of use of jewelry featuring such a body.

It has been found that balsa wood, which is obtained from the *ochroma lagopus* or *pyramidale* is well suited for this purpose. The porosity of balsa wood allows for nearly uniform absorption throughout the whole body of ligneous material of many types of alcohol based volatile liquids.

It is known that moisture induces and/or promotes decay of wood. If balsa wood is treated with an impregnating agent, e.g. *colophony* and/or *paraffin wax*, prior to serving its purpose as a perfume absorber, then the harmful effects of oxidation or decay can be prevented, extending the operational lifespan of the material. Therefore, according to an embodiment there is provided a method of manufacturing the piece of jewelry comprising the step of treating the body of porous material 6 with an impregnating agent, prior to use.

Due to its rigid structure, balsa wood does not bulge, flocculate or pulverize, even if exposed to humid environments. Moist balsa wood retains its shape, representing a reliable material that will not get stuck inside the rigid casing 4, or obstruct or disperse through the recesses provided for perfume emission in the piece of jewelry.

The rigid casing 4 is composed of materials that enable the piece of jewelry to retain its designed shape. Basically any solid material may serve this purpose, although restrictions are assumed to be posed by material properties like toxicity or radioactivity that do not allow the piece of jewelry to be used as such. In an embodiment, the perfume emitting jewelry may be manufactured from non-ferrous alloys, with relatively low production costs. In a subsequent production stage, the exterior of the rigid casing 4 may be coated with precious metal, contributing to the aesthetic appeal. Gold, silver and platinum represent obvious coating materials. In another embodiment, the rigid casing 4 may also consist entirely of precious metals, requiring more substantial production resources. A piece of jewelry may also be constructed from non-metallic materials. Materials with a low mass density may prove beneficial for pieces of jewelry for which a low weight yields an increased ease of use, e.g. for earrings. Obviously, the piece of jewelry may be realized in various designs. It may be designed as a pendant, a necklace, a bracelet, a broche or an earring. Examples of these will be explained in more detail below with reference to the figures.

### Aerodynamic considerations

In order to achieve an optimal emission of the liquid fragrance, the body of porous material, the casing and the slots are designed in a balanced way. The perfume emission mechanism is based on the body temperature of a user, causing the perfume contained by the body of porous material 6 to evaporate. The piece of jewelry may be designed with a rigid casing 4 that has an elongated exterior shape along a body axis A. In order to transport the perfume to the surroundings, one or more slots 10 in the rigid casing are provided. At least one of these slots 10 constitutes an aerial duct between the cavity 5 and the surroundings, allowing the perfume to diffuse from the porous body into the surrounding air and/or allowing convection to transport the perfume out of the cavity into the surroundings. By optimizing the size, shape and location of the slots 10, a desired rate of perfume emission can be obtained. Apart from contributing to the emission characteristics, another benefit of the slots 10 is that they provide sufficient exposure of the ligneous porous body to air, suppressing decay of the moisture saturated ligneous material.

The aerodynamic properties of such a configuration of slots are not restricted to pieces of jewelry with a porous body of ligneous material in general, or balsa wood in particular, but also pertain to pieces of jewelry with a body of porous material consisting of different materials.

According to an embodiment, the piece of jewelry comprises at least one slot 10 which has a substantially elongated shape along a surface of the rigid casing 4. The slots 10 are an elongated type of recess, with the elongated shape formed by two substantially parallel edges that mark the interface between the rigid casing 4 and the recess. This elongation defines a direction that is tilted at a certain angle with respect to the body axis A. Each slot 10 has a width 16 defined by its perpendicular shortest distance between the substantially parallel edges. The width 16 of the slot may be in the range of 0.5 - 2.0 mm. It has been experimentally found that a width smaller than 0.5 mm prevents the air from effectively circulating. A width of over 2 mm may in principle be provided, but slots of larger size tend to expose too large a portion of the casings innards, that is the porous body, which detracts from the aesthetic properties of the piece of jewelry. In an embodiment, a width of 0.5 - 0.6 mm has been proven to provide a balanced emission of perfume, i.e. not too much or too little perfume per time unit.

Furthermore, an interspacing 14 of 0.1 - 0.2 mm may be provided between the interior surface of the rigid casing 4 and the body of porous material 6, when saturated with the liquid fragrance. As an advantage, a larger part of the surface of the porous body is exposed to air, such that a balanced amount of perfume is emitted per time unit. Furthermore, the force of friction between the porous body and the casings interior surface is kept to a minimum, allowing for easy extraction without chance of damaging the porous body.

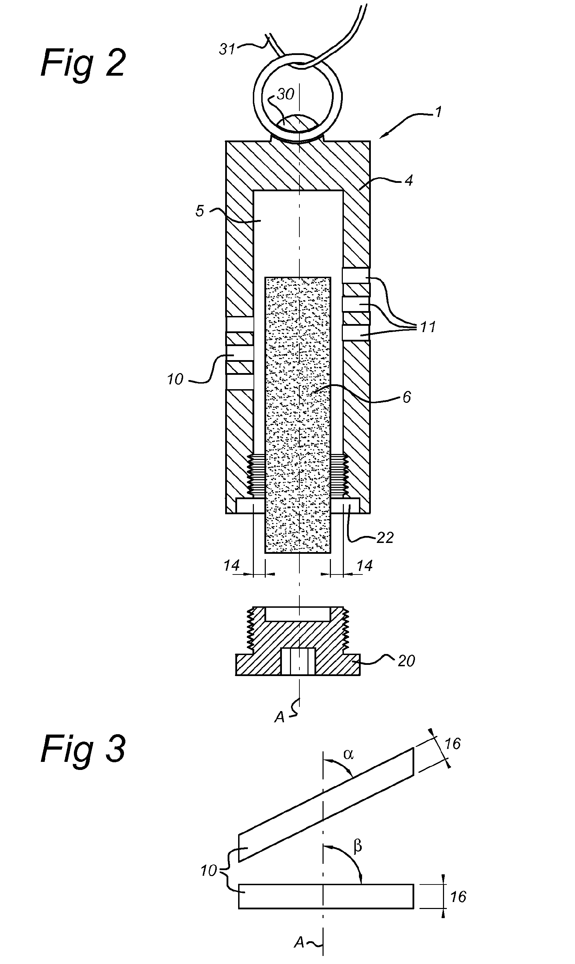

Fig. 3 shows the orientation of a pair of slots 10 with respect to the body axis A.
The elongation of each slot 10 is substantially parallel to the surface of the casing 4, defining a direction that is tilted at a specified angle with respect to the body axis A. In an embodiment, multiple similarly oriented slots are grouped into pluralities of slots 11, 12, with similar angles between the direction of elongation of each slot and the body axis A. As a result, all slots in each plurality are locally parallel. Fig. 3 illustrates one slot 10 in the first plurality of slots 11 that is oriented at a first angle α with respect to the body axis A.

According to an embodiment, the orientation of the first plurality of slots 11 may be substantially diagonal with respect to the body axis A, with the first angle α assumed to be within the range of 30° to 60°. The diagonal character of the slots serves to guide the air that is caused to rise up due to the generated body heat along the exposed surface of the porous body. In one embodiment, an angle of 45° is selected for slots that extend along a major part of the casing surface, maximizing the length of the slots and the exposed surface of the porous body without compromising the structural integrity of the rigid casing 4.

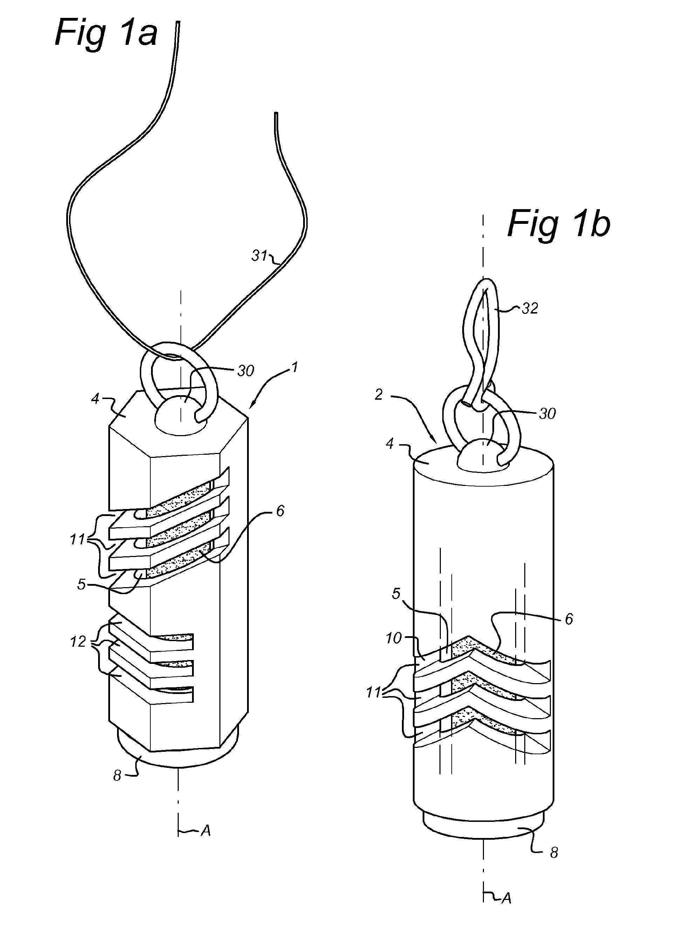

According to an embodiment, a second plurality of slots 12 is provided, wherein each slot 10 of the second plurality of slots 12 is oriented at a second angle β with respect to the body axis A, the second angle possibly differing from the first angle α. It is possible for the perfume to persistently accumulate in a specific region of the porous body that is not sufficiently exposed to air. As an example, for an embodiment with a linear body axis A that, while in use, is substantially parallel to the direction of gravity, e.g. for a piece of jewelry designed as a pendant (Fig. 1a), the perfume is inclined to sink to the lower part of the porous body due to the gravitational force. In order to prevent persisting accumulation of perfume in specific regions that are not exposed to air, the second plurality 12 of slots is provided near the location of accumulation.

In an embodiment of a perfume-emitting six-sided right prism pendant 1, the first and second pluralities of slots 11, 12 each consist of three slots with a width 16 of 0.5 - 0.6 millimeters. The casing 4 of the pendant has a length of 25 - 30 millimeters and a width of 9 - 10 millimeters. The first plurality of slots 11 is tilted at an angle α substantially equal to 45 degrees with respect to the body axis A. The second plurality of slots 12 is tilted at an angle β of 90 degrees with respect to the body axis and may be located at the bottom of the rigid casing. The ligneous porous body 6 is a cylindrical unit of balsa wood with a height of 20 mm and a radius of 3 millimeters. For this embodiment in an ambient temperature of 18° to 25°, it has been established by experiment that the evaporation of a single perfume filling will last up to three to four weeks.

### Means of attachment

In general, wearing jewelry requires a means of attachment preferably for non-invasive fixation to the person's body. For the piece of jewelry currently described, the means of attachment may be selected from a set consisting of bands, chains, rings, ear clips, ear posts, or ear wires.

Embodiments will now be described more fully hereinafter with reference to the accompanying drawings, in which some examples of the embodiments are shown. Like reference symbols refer to like elements throughout.

### Pendant or earring

According to an embodiment there is provided a piece of jewelry, wherein the piece of jewelry is a pendant 1 or an earring 2 in which, when worn, the first body axis A is substantially parallel to the direction of the gravitational force. Examples of these are explained in more detail with reference to Fig.'s 1a, 1b and 2.

Fig.'s 1a and 1b schematically show perspective views of embodiments of perfume-emitting jewelry. In the embodiment of Fig. 1a, the piece of jewelry is designed as a six-sided right prism pendant 1. Fig. 1b illustrates a piece of jewelry shaped as a cylindrical earring 2. All embodiments comprise a casing of rigid material 4, which forms a cavity 5. This cavity is arranged for holding the body of porous material 6 suitable for absorbing and emitting perfume. This cavity is sealed off by an opening and closing mechanism 8. One or more slots 10 are provided, of which several are grouped into pluralities of slots 11, 12. A means of attachment is provided, comprising a knob 30 on top of the rigid casing 4, and a means of fixation to the person's body 31, 32, optionally including a loop.

In addition to holding the piece of porous material 6, the rigid casing 4 defines the exterior shape of the piece of jewelry, and therefore may serve an aesthetic purpose. In an embodiment, the exterior shape of the casing 4 is substantially elongated, the direction of elongation corresponding to the body axis A.

The piece of jewelry in Fig.'s 1a and 1b represent a pendant and an earring respectively. This requires a means of attachment 30, 31, 32 preferably for non-invasive fixation to the person's body. For both the pendant and the earring the means of attachment comprises a knob 30 for attaching the means of fixation to the person's body 31, 32 to the casing 4. The means of attachment in the embodiment of a pendant show in Fig. 1a furthermore comprises a loop and a chain 31 which enables the pendant to be worn around the neck. As alternatives to a chain, wires or bands would equally suffice. The means of attachment in the embodiment of an earring show in Fig. 1b may be an ear hook 32, an ear wire, an ear clip, or an ear post.

In an embodiment, the casing exterior may be an elliptic cylinder or a right n-prism, were n is a positive integer greater than 2, which is symmetrical with respect to the body axis A. This symmetry facilitates the production process, reducing the amount of operations executed by production machinery. In another embodiment, the cavity 5 may be arranged to have a cylindrical shape, which further facilitates production.

Fig. 2 presents a schematic cross-section of an embodiment of a perfume-emitting six-sided right prism pendant 1. The cavity 5 inside the rigid casing 4 offers space for the porous body 6 suitable for containing perfume. An interspacing 14 is provided between the inside of the rigid casing 4 and the outer surface of the porous body 6, when inserted into the cavity 5. The interspacing 14 may be in the range of 0.1 - 0.2 mm.

In this embodiment, the opening and sealing mechanism 8 comprises a cap 20, which can be removed to create an opening 22, which opening and cap are arranged to seal off the opening, allowing the body of porous material 6 to be removed from and inserted into the cavity 5. The opening and closing mechanism 8 for sealing off the cavity is arranged to completely seal off an opening 22, to prevent leaking of liquid perfume.

As described above, one or more slots 10 are provided. Three slots 10 are grouped in a first plurality of slots 11, having an angle α in the range of 30 - 60 degrees with respect to the body axis A. Three other slots 10 are grouped in a second plurality of slots 12, having an angle β of substantially 90 degrees with respect to the body axis A.

A means of attachment is provided, comprising a knob 30 on top of the casing 4, together with a hoop and a chain 31 enabling the pendant to be worn around a person's neck.

The porous body 6 can easily be removed from the piece of jewelry and can easily be refilled by spraying it with perfume or by immersion into a reservoir containing liquid perfume. An advantage of the removable porous body 6 is that the perfume is easily replenished or replaced, while the possibility of spilling the liquid perfume through the recesses 10 or other openings is eliminated. Furthermore, the porous body 6 impregnated with liquid perfume eliminates the need for a separate reservoir containing the perfume in liquid state. The porous body 6 may consist of ligneous material and balsa wood in particular.

Durability and ease of use of the piece of jewelry is further increased if the opening and closing mechanism 8 required for replenishing or replacing the body of porous material is robust and less prone to wear. The rigid casing 4 contains an opening 22 for inserting or removing the porous body. In an embodiment, a cap 20 is arranged to seal off the opening. The means of attaching the cap 20 to the opening 22 may be a hex- or torx-key based screwing mechanism, fastening and removing constituents by twisting motion. Obviously, other opening and sealing mechanisms known to the person skilled in the art may be provided.

### Bracelet

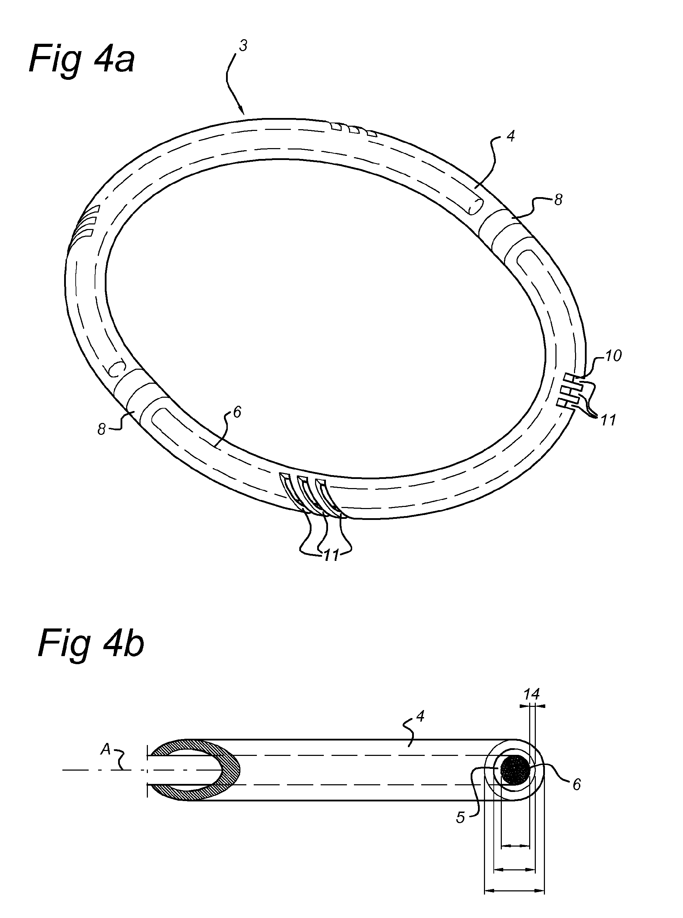

According to a further embodiment, the piece of jewelry is a bracelet 3 wherein the first body axis A is substantially circular or helical. Fig. 4a schematically shows such an embodiment, in which the piece of jewelry assumes the form of a toroidal bracelet 3. Fig. 4b depicts an exploded view of this bracelet. More generally, in this embodiment the exterior of the casing 4 can be considered to be ring-shaped. The ring-shaped embodiment has a circular or helical body axis A. The cavity 5 arranged for holding the porous body of ligneous material 6 is also shaped as a helix or a ring, similar to the exterior shape of the casing 4. As the body of porous material 6 is not arranged to be removable, no interspacing between the body of porous material 6 and the casing inner surface is required, although may be present.

According to an embodiment, the opening and sealing mechanism 8 comprises a sliding cap and a tap hole, both not shown in the figures. The tap hole allows refilling of the body of porous material 6 by pouring perfume through the tap hole, while the sliding cap 26 is arranged for sealing off the tap hole. In this embodiment, the slots 10 enabling air circulation are also grouped into pluralities of slots 11. Three slots 10 may be grouped in a first plurality of slots 11, having an angle α in the range of 30 - 60 degrees with respect to the body axis A. as can be seen in Fig. 4a, more than one first plurality of slots 11 may be provided.

A ring-shaped piece of jewelry may also represent an earring, which could be considered a small equivalent of a toroidal bracelet. This embodiment requires a portion of the ring to be removed and to be substituted for a means of attachment to the person's ear. If the ear is already perforated, this means of attachment may be an ear wire, an ear pin or an ear post. In absence of a perforation, an ear clip would present an alternative means of attachment.

It will be understood that in the embodiment shown in Fig.'s 4a and 4b the body of porous material 6 is curved along at least part of the circular or helical body axis A. Processing and employing ligneous materials like balsa wood in combination with a curved cavity 5 has been found to be difficult. A porous body of balsa wood will easily fracture if too much pressure or tension is exercised during insertion or removal. As the insertion of the body of porous material 6 into the curved cavity 5 has to occur at least once, a method has been developed for fixating the body of porous material 6 during the manufacturing process. This method for permanently fixating the body of porous material 6 inside the piece of jewelry comprises the following steps:

Prior to insertion into the cavity 5, the body of porous material 6 is soaked with a volatile liquid. Once the body of porous material is saturated with this volatile liquid, the body is inserted carefully through the tap hole into the cavity 5. This is now possible without fracturing the wood as the body of porous material is more flexible when saturated with liquid. Once the body of porous material is in place, this embodiment of the piece of jewelry is exposed to the ambient for an interval of time required for the volatile liquid to evaporate and the porous body to become depleted. After this time interval has elapsed, refilling the fixed body of porous material is possible by pouring perfume through the tap hole. So, there is provided a method for fixating the body of porous material 6 inside the cavity 5 of the piece of jewelry having a first body axis A that is substantially circular or helical, the method comprising:
(a) soaking the body of porous material 6 with volatile liquid prior to insertion into the cavity 5;
(b) inserting the soaked body of porous material through the tap hole into the cavity.

The liquid substance used in (a) may be water or the like, only used for the purpose of fixating the body of porous material 6 inside the cavity 5. However, this liquid may also be the liquid fragrance. In the latter case, the prepared piece of jewelry should be ready for use, if packaged under sufficient sealing conditions.

If the liquid is not the liquid fragrance, further actions (c) and (d) may be performed:
(c) exposing the piece of jewelry with the inserted body of porous material to the ambient for a predetermined time interval, such that the porous body has become depleted.
   The piece of jewelry is now ready for use. The method may further comprise:
(d) refilling the fixed body of porous material by supplying liquid fragrance through the tap hole.

It will be apparent to the person skilled in the art that other alternative and equivalent embodiments of the invention can be conceived and reduced to practice without departing from the spirit of the invention, the scope of the invention being limited only by the claims set out below.

## Claims

1. Piece of jewelry (1,2,3), for use on a person's body, comprising a rigid casing (4) forming a cavity (5), the cavity being arranged to hold a body of porous material (6) suitable for absorbing and emitting a liquid fragrance, the casing comprises an opening and sealing mechanism (8) allowing the body of porous material inside the cavity to be accessed, and the casing comprises at least one slot (10), the at least one slot constituting an aerial duct between the cavity and the surroundings of the piece of jewelry, allowing the liquid fragrance to evaporate, **characterized in that**
- the body of porous material consists of a ligneous absorbent.

2. Piece of jewelry according to claim 1, wherein the body of porous material (6) consists of balsa wood, which is obtained from the *ochroma lagopus* or *pyramidale.*

3. Piece of jewelry according to claim 1, wherein the rigid casing (4) has an elongated exterior shape along a body axis (A).

4. Piece of jewelry according to any one of the preceding claims, wherein an interspacing (14) of 0.1 - 0.2 mm is provided between the interior surface of the rigid casing (4) and the body of porous material (6), when saturated with the liquid fragrance.

5. Piece of jewelry according to any one of the preceding claims, wherein the at least one slot (10) has a substantially elongated shape along a surface of the rigid casing (4) and a width (16) in the range of 0.5 - 2.0 mm.

6. Piece of jewelry according to any one of the claims 3 - 5, comprising a first plurality of slots (11), wherein each slot (10) of the first plurality of slots is oriented at a first angle (α) with respect to the body axis (A).

7. Piece of jewelry according to claim 6, wherein the first angle (α) is in the range of 30° - 60°.

8. Piece of jewelry according to any one of the claims 3 -7, comprising a second plurality of slots (12), wherein each slot (10) of the second plurality of slots is oriented at a second angle (β) with respect to the body axis (A), the second angle differing from the first angle (α).

9. Piece of jewelry according to claim 8, wherein the second angle (β) is substantially equal to 90°.

10. Piece of jewelry according to any one of the preceding claims, wherein the piece of jewelry is a pendant (1) or an earring (2) in which, when worn, the body axis (A) is substantially parallel to the direction of the gravitational force.

11. Piece of jewelry according to any one of the preceding claims, wherein the opening and sealing mechanism (8) comprises a cap (20), which can be removed to create an opening (22), which opening and cap are arranged to seal off the opening, allowing the body of porous material (6) to be removed from and inserted into the cavity (5).

12. Piece of jewelry according to any one of the claims 1 - 9, wherein the piece of jewelry is a bracelet (3) wherein the body axis (A) is substantially circular or helical.

13. Piece of jewelry according to claim 12, wherein the body of porous material (6) is curved along at least part of the circular or helical body axis (A).

14. Piece of jewelry according to claim 12, wherein the opening and sealing mechanism (8) comprises a sliding cap for closing off a tap hole that allows refilling of the body of porous material (6) by pouring perfume through the tap hole.

15. Piece of jewelry according to any one of the preceding claims, wherein the body of porous material (6) has been treated with an impregnating agent.

16. Method for fixating the body of porous material (6) inside the cavity (5) of the piece of jewelry according to claims 12 - 14, the method comprising:
(a) soaking the body of porous material (6) with a volatile liquid prior to insertion into the cavity (5);
(b) inserting the soaked body of porous material through the tap hole into the cavity.

17. Method according to claim 16, the method further comprising
(c) exposing the piece of jewelry with the inserted body of porous material (6) to the ambient for a predetermined time interval, such that the porous body has become depleted.

18. Method according to claim 17, wherein the method further comprises:
(d) refilling the inserted body of porous material (6) by supplying liquid fragrance through the tap hole.
